# EUROPEAN PATENT APPLICATION

(11) **EP 4 702 913 A1**
(43) Date of publication of application: **04.03.2026**
(21) Application number: 24197406.2
(22) Date of filing: 29.08.2024
(51) Int. Cl.: A61B 1/00, A61B 1/045, A61B 1/06, A61B 1/24, A61B 5/00, G16H 20/40, G16H 40/63

(54) **SMART DENTAL EXAMINATION DEVICE**

(71) Applicant: Koninklijke Philips N.V., 5656 AG Eindhoven (NL)
(72) Inventor: JOHNSON, Mark Thomas, Eindhoven (NL); GERHARDT, Lutz Christian, Eindhoven (NL); VAN DEN DUNGEN, Wilhelmus Andreas Marinus Arnoldus Maria, Eindhoven (NL)
(74) Representative: Philips Intellectual Property & Standards

(57) **Abstract**

A dental examination system is presented, comprising: an imaging system for recording a dental image; a light source ; a processor configured to: receive data containing information on a dental related problem; select for the dental related problem a predetermined setting from a set of settings for the imaging system and/or the light source, each setting optimized for imaging a specific dental-related problem; configure the imaging system and/or light source with the selected predetermined setting; recording a dental image using the configured imaging system and/or light source . A method for recording a dental image is also presented.

## Description

### FIELD OF THE INVENTION

The present disclosure relates to dental examination technologies, specifically to smart dental mirrors with adaptive illumination systems for optimized imaging.

### BACKGROUND OF THE INVENTION

Dental professionals face significant challenges in accurately diagnosing and treating various dental conditions. Traditional dental examination tools often lack the capability to provide detailed and specific imaging required for precise diagnosis. This limitation can lead to prolonged examination times and potential misdiagnosis, impacting the overall efficiency and effectiveness of dental care.

Existing solutions, such as standard dental mirrors and basic imaging systems, do not offer the flexibility to adapt to different dental conditions. These tools typically use a single wavelength of light for illumination, which may not be optimal for detecting specific dental issues like caries, gum inflammation, or plaque. Additionally, continuous use of certain illumination frequencies, such as blue or UV light, can pose safety risks and result in the generation of large, unmanageable data sets.

### SUMMARY OF THE INVENTION

Particular and preferred aspects of the invention are set out in the accompanying independent and dependent claims. Features from the dependent claims may be combined with features of the independent claims and with features of other dependent claims as appropriate and not merely as explicitly set out in the claims.

These and other aspects of the invention will be apparent from and elucidated with reference to the embodiment(s) described hereinafter.

According to an aspect of the present invention, a dental examination system comprises an imaging system for recording a dental image, a light source, and a processor configured to receive data containing information on a dental-related problem, select for the dental-related problem a predetermined setting from a set of settings for the imaging system and/or the light source, each setting optimized for imaging a specific dental-related problem, configure the imaging system and/or light source with the selected predetermined setting, and record a dental image using the configured imaging system and/or light source.

According to another aspect, the system receives the data containing information on a dental-related problem from a source external to the system and provides it to the processor.

According to yet another aspect, receiving data containing information on a dental-related problem comprises receiving an image and processing the image to determine the dental-related problem from the image. The image may be an image taken from tooth material from a subject.

According to another aspect, the set of settings comprises wavelength selection and/or filter characteristics of the imaging system and/or light source.

According to yet another aspect, the system further comprises an oral healthcare device configured to generate the data containing information on a dental-related problem. The device may be a toothbrush, an oral irrigator or another device capable of generating data containing information on a dental-related problem.

According to another aspect, the system is a portable dental examination instrument comprising a mirror and the processor is further configured to select the predetermined setting based on location and/or orientation of the mirror, preferably relative to a tooth surface.

According to yet another aspect, the mirror is a dental examination mirror which integrates the imaging system and the light source.

According to another aspect, the processor is configured to adapt other settings of the imaging system and/or the light source such as auto-zoom, contrast, frame rates, resolution, and light intensity, based on the data received.

According to yet another aspect, a method for recording a dental image comprises receiving data containing information on a dental-related problem, selecting for the dental related problem a predetermined setting from a set of settings for an imaging system and/or a light source, each setting optimized for imaging a specific dental-related problem, configuring the imaging system and/or light source with the selected predetermined setting, and recording a dental image using the configured imaging system and/or light source.

According to another aspect, the method further comprises receiving the data containing information on a dental-related problem from a source external to the smart dental mirror system.

According to yet another aspect, receiving data containing information on a dental-related problem comprises receiving an image and processing the image to determine the dental-related problem from the image. The image may be an image taken from tooth material from a subject.

According to another aspect, the method further comprises selecting the predetermined setting based on wavelength selection and/or filter characteristics of the imaging system and/or light source.

According to yet another aspect, the method further comprises selecting the predetermined setting based on the location and/or orientation of a mirror relative to a tooth surface, wherein the mirror integrates the imaging system and the light source.

According to another aspect, the method further comprises adapting other settings of the imaging system and/or light source, including auto-zoom, contrast, frame rates, resolution, and light intensity, based on the received data.

According to yet another aspect, the method is implemented in the processor of the dental examination system as described above.

### BRIEF DESCRIPTION OF THE DRAWINGS

The drawings are only schematic and are non-limiting. In the drawings, the size of some of the elements may be exaggerated and not drawn on scale for illustrative purposes.

Any reference signs in the claims shall not be construed as limiting the scope.

In the different drawings, the same reference signs refer to the same or analogous elements.
FIG 1 illustrates a box diagram of an embodiment of the invention.
FIG 2 illustrates a box diagram of an embodiment of the invention.
FIG 3 illustrates a dental examination device, wherein the light source and imaging system are integrated in a dental examination mirror.
FIG 4 illustrates a box diagram of a method for recording a dental image.
FIG 5 illustrates a box diagram of a method for recording a dental image.

### DETAILED DESCRIPTION OF THE EMBODIMENTS

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

Furthermore, the terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In the description provided herein, numerous specific details are set forth. However, it is understood that embodiments of the invention may be practiced without these specific details. In other instances, well-known methods, structures and techniques have not been shown in detail in order not to obscure an understanding of this description.

The described dental examination system addresses the problems identified in the background of the invention section by presenting an advanced dental examination system that includes an imaging system, a light source, and a processor. The processor receives data containing information on a dental-related problem and selects a predetermined setting from a set of settings for the imaging system and/or the light source. Each setting is optimized for imaging a specific dental-related problem. The system configures the imaging system and/or light source with the selected predetermined setting and records a dental image using the configured components. This approach ensures that the dental examination is both safe and efficient, reducing the time required for image acquisition and minimizing data volume. Further, as the system is pre-programmed with optimal settings for various dental conditions, allowing it to automatically select the most settings based on the specific dental issue being examined; this reduces the need for manual adjustments and enhances diagnostic accuracy.

The described system addresses the issue of minimizing data volume through several key mechanisms. By pre-programming the system with the most suitable settings for the most likely dental problem, the system avoids the need to capture images at multiple wavelengths continuously. This selective use of wavelengths reduces the amount of data generated, as only the most relevant wavelengths are used for imaging specific dental issues. Further, the processor selects predetermined settings optimized for imaging specific dental-related problems. This means that the imaging system and light source are configured to capture the most relevant and high-quality images for the identified problem, reducing the need for multiple images and thereby minimizing data volume. Further, by configuring the imaging system and light source with the selected predetermined settings, the system ensures that the dental examination is efficient. This reduces the time required for image acquisition, as the system is already optimized for the specific dental problem, leading to fewer images being taken and less data being generated. Further, as the system can receive data from external sources, such as home oral healthcare devices or electronic medical records. This prior knowledge allows the system to focus on the most likely dental problems, further reducing the need for extensive imaging and data collection. Thus, the dental examination system effectively minimizes the volume of data generated during dental examinations, addressing the issue of data reduction while maintaining high diagnostic accuracy and efficiency.

In a first aspect of the invention, a dental examination system 100 is presented, comprising: an imaging system 101 for recording a dental image; a light source 102; a processor 103 configured to: receive data containing information on a dental related problem; select for the dental related problem a predetermined setting from a set of settings for the imaging system 101 and/or the light source 102, each setting optimized for imaging a specific dental-related problem; configure the imaging system 101 and/or light source 102 with the selected predetermined setting; recording a dental image using the configured imaging system 101 and/or light source 102. The dental examination system 100 aims to improve the accuracy of dental diagnoses, reduce examination times, and enhance overall dental care.

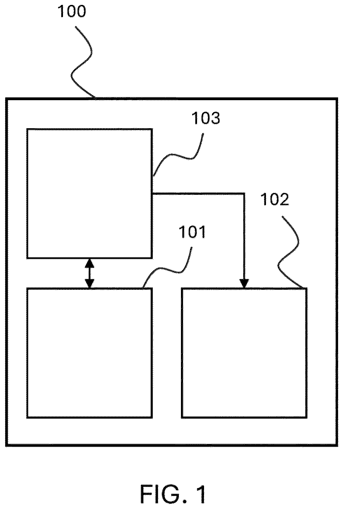

FIG 1 illustrates an embodiment of a dental examination system 100. A dental examination system 100 contains a processor 103. The processor 103 controls a light source 102 and an imaging system 101. The light source 102 may be a light source 102 capable of emitting light in different wavelength ranges. The light source 102 may be configurable ensuring that the light source 102 is capable of emitting light of different wavelengths or different wavelength ranges.

The configuration of the imaging system 101 and/or light source 102 comprises setting parameters for the imaging system 101 and/or light source 102. According to different embodiments these parameters may include:
- Wavelength Selection: The specific wavelength or range of wavelengths of light emitted by the light source 102. For example, selecting a wavelength of 520 nm for imaging enamel lesions and caries.
- Filter Characteristics: The type and characteristics of optical filters used in the imaging system 101 to enhance the detection of specific dental conditions. For instance, using a filter that allows only light of 630 nm to pass through for detecting plaque and tartar.
- Light Intensity: The brightness or intensity of the light source 102, which can be adjusted to optimize the illumination for different dental conditions and to ensure patient safety.
- Auto-Zoom: The zoom level of the imaging system 101, which can be automatically adjusted to focus on specific areas of interest within the oral cavity.
- Contrast: The contrast settings of the imaging system 101, which can be modified to enhance the visibility of certain dental features, such as caries or gum inflammation.
- Frame Rates: The rate at which images are captured by the imaging system 101, which can be adjusted to balance image quality and data volume.
- Resolution: The resolution of the images captured by the imaging system 101, which can be set to provide the necessary level of detail for accurate diagnosis.
- Duty Cycle of Modulation: For pulsed light sources, the duty cycle of modulation can be adjusted to control the duration and frequency of light pulses, optimizing the imaging process for different dental conditions.
- Imaging Modes/Modalities: The selection of different imaging modes or modalities, such as switching between reflectance and fluorescence measurements, to capture the most relevant images for specific dental problems.

By configuring these parameters, the dental examination system can be tailored to provide optimal imaging for a wide range of dental conditions, ensuring accurate diagnosis and efficient examination processes.

The imaging system 101 may be an imaging component such as an image sensor capable of recording light in different wavelength ranges. The image sensor may be a hyperspectral image sensor which allows the imaging system to capture detailed images across a wide range of wavelengths. This capability may be advantageous for accurately diagnosing various dental conditions, as different dental issues are better visualized at specific wavelengths. For instance, caries may be more visible at 520 nm, while other conditions like gum inflammation or plaque might require different wavelengths for optimal imaging. By using a hyperspectral image sensor, the dental examination system 100 can adapt to these varying requirements, ensuring that the most relevant and detailed images are captured for each specific dental problem.

The processor 103 controls the light source 102 and the imaging system 101 based on data received containing a dental problem. The data is used to select which settings of the imaging system 101 and/or light source 102 are most optimal for imaging the dental problem. The settings are retrieved from a set of predetermined settings for the imaging system 101 and/or the light source 102. Each of these settings are predetermined for a particular dental related problem. Once the processor103 received the information/data on the dental related problem, the particular settings for the imaging system 101 and/or light source 102 are extracted from the set of predetermined settings and used to configure the imaging system 101 and/or the light source 102. The selection may be performed from a look-up table containing the predetermined settings. Once the imaging system 101 and/or light source 102 are configured, the imaging system 101 and/or light source 102 are used to record an image. The light source 102 is activated timely ensuring that imaging using the imaging system 101 can be done.

According to an embodiment, the imaging system 101 is a reconfigurable imaging system of which light filter characteristics can be configured real-time. According to an embodiment the light source 102 is a reconfigurable light source of which the emitted light characteristics can be configured real-time.

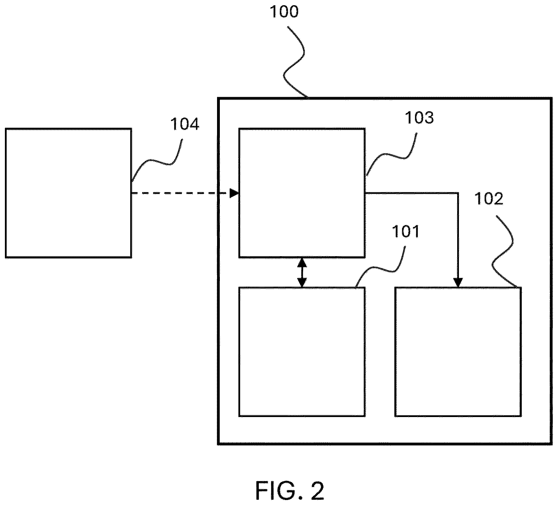

FIG 2 illustrates an embodiment of the dental examination system 100. In this embodiment, the processor 103 receives data from a source 104 external to the dental examination system 100.

According to different embodiments, these sources could be:
- Electronic Medical Records (EMR) Systems: Data from a patient's medical, e.g. dental, history, including previous dental images, diagnoses, and treatment plans, can be accessed from EMR systems to inform the current examination.
- Home Oral Healthcare Devices: Data collected from devices such as smart toothbrushes or home dental cameras, which monitor and record oral health conditions over time, can be transmitted to the dental examination system 100.
- Cloud-Based Health Platforms: Health data stored on cloud platforms, including patient-reported symptoms and remote monitoring data, can be integrated into the dental examination system for a comprehensive view of the patient's oral health.
- Wearable Health Devices: Information from wearable devices that track health metrics such as heart rate, stress levels, and other physiological parameters can provide additional context for dental examinations.
- Laboratory Test Results: Data from laboratory tests, such as saliva analysis or microbiome studies, can be used to identify potential dental issues and guide the examination process.
- Telehealth Consultations: Data from remote consultations with dental professionals, including images and notes taken during virtual visits, can be incorporated into the examination system 100.
- Patient Self-Reports: Information provided directly by the patient, such as pain levels, symptoms, and oral hygiene practices, can be used to tailor the examination settings.
- Dental Imaging Archives: Historical dental images stored in digital archives can be referenced to compare current conditions with past records, aiding in the diagnosis and treatment planning.

By utilizing data from these external sources, the dental examination system 100 can enhance its diagnostic capabilities and provide more personalized and accurate dental care.

According to an embodiment, the dental examination system 100 receiving data containing information on a dental related problem comprises: receiving the dental image; and processing the dental image to determine the dental related problem. The processing of the dental image may comprise different techniques such as:
- Image Segmentation and Classification: The system 100 can use advanced image segmentation techniques to isolate different regions of the dental image. For instance, the system can segment the image into regions representing teeth, gums, and other oral structures. Once segmented, machine learning algorithms, such as convolutional neural networks (CNNs), can classify these regions to identify specific dental problems like caries, gum inflammation, or plaque.
- Spectral Analysis: The system 100 can utilize spectral analysis to identify dental problems based on the specific wavelengths of light reflected or absorbed by different dental tissues. Different dental conditions exhibit unique spectral signatures, which can be detected and analyzed to determine the problem.
- Machine Learning and AI: Machine learning models can be trained on large datasets of dental images to recognize patterns and features indicative of various dental problems. These models can then be used to analyze new images and identify potential issues.
- Texture Analysis: Texture analysis techniques can be used to identify irregularities in the surface texture of teeth and gums, which may indicate dental problems. For example, caries often present as rough or pitted areas on the tooth surface.
- Fluorescence Imaging: Fluorescence imaging can be used to detect dental problems based on the fluorescence properties of dental tissues. For example, caries and plaque fluoresce differently under specific wavelengths of light.

By integrating these technical approaches, the dental examination system 100 can effectively process dental images to determine the presence and type of dental-related problems, thereby enhancing diagnostic accuracy and improving patient care.

According to an embodiment of this disclosure, the dental examination system 100 comprises a mirror 105 for allowing a dentist to perform examination of teeth and oral cavity.

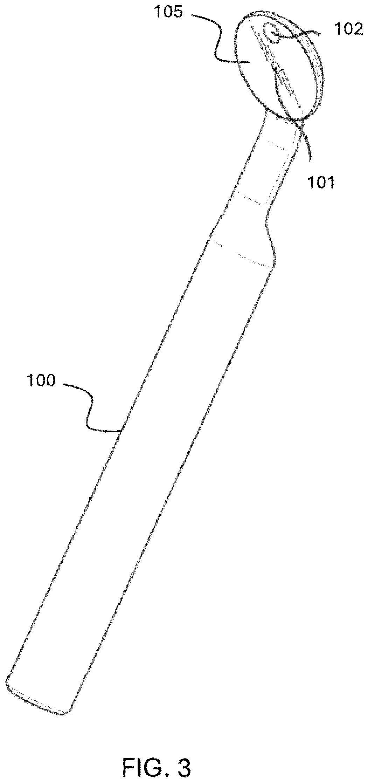

FIG. 3 shows another embodiment of a dental examination system 100. The dental examination system 100 incorporates an imaging system 101 and a light source 102. The imaging system 101 and the light source 102 are integrated into a mirror 105. The dental examination system 100 provides a handheld device that facilitates dental examinations. The imaging system 101 captures images of the teeth and surrounding areas, allowing for detailed inspection and diagnosis. The light source 102 illuminates the area being examined. The mirror 105 serves a dual purpose by reflecting the image of the teeth while also housing the imaging system 101 and the light source 102. This integration allows the dentist to use a single tool for both visual examination and image capture, enhancing efficiency during dental procedures.

To integrate the imaging system 101 and the light source 102 into the mirror 105, several technical considerations and steps can be taken. Example of a dental examination tool with mirror and integrated electronics:
The imaging system 101 and the light source 102 need to be miniaturized to fit within the limited space of the mirror 105. This can be achieved by using compact camera modules and LED light sources. For example, a small CMOS or CCD sensor may be used for the imaging system 101. High-intensity, low-power LEDs may be used for the light source 102.The mirror 105 can be designed with cavities or slots to house the imaging system 101 and the light source 102. The components can be securely mounted within these cavities. The imaging system 101 and the light source 102 require electrical connections for power and data transmission. These connections can be routed through the handle of the dental examination system 100. It should be ensured that the imaging system 101 and the light source 102 are optically aligned to provide clear and well-illuminated images. The light source 102 should be positioned to avoid glare and reflections on the mirror 105. For example, the light source 102 is positioned at an angle to the mirror 105 to provide even illumination without causing reflections. In an embodiment, optical lenses or diffusers are used to direct and spread the light from the light source 102. The processor 103 may be located in the handle and is running the software used to control the imaging system 101 and the light source 102.

In an aspect of the disclosure, the processor 103 is further configured to select the predetermined setting based on location and/or orientation of the mirror 105, preferably relative to a tooth surface.

In an aspect of the disclosure, the processor 103 is further configured to select the predetermined setting based on the location and/or orientation of the mirror 105, preferably relative to a tooth surface. This is important for several reasons:
The location and orientation of the mirror 105 relative to the tooth surface can affect how well the area is illuminated. By adjusting the light source 102 based on the mirror's position, the system can ensure that the tooth surface is evenly and adequately lit, reducing shadows and enhancing visibility.

### Example:

In an embodiment, if the mirror 105 is positioned at an angle, the light source 102 may be adjustable to direct light more effectively onto the tooth surface. The processor 103 may increase the brightness of the light source 102 when the mirror 105 is further away from the tooth surface to compensate for the increased distance.

The orientation of the mirror 105 can impact the quality of the images captured by the imaging system 101. By selecting settings based on the mirror's 105 position, the system can optimize the imaging parameters to capture clearer and more detailed images.

### Example:

In an embodiment, the processor 103 may be able to adjust the focus and exposure settings of the imaging system 101 based on the angle and distance of the mirror 105 from the tooth surface. The processor 103 may apply image stabilization techniques if the mirror 105 is in a less stable position, ensuring that the captured images are sharp and free of motion blur.

The ability to adjust settings based on the mirror's 105 location and orientation allows for more accurate diagnosis of dental issues. Different angles and positions can reveal different aspects of the tooth surface, and optimizing the settings for each position ensures that all relevant details are captured.

### Example:

In an embodiment, the processor 103 may switch to a higher resolution mode when the mirror 105 is positioned to capture a close-up image of a specific area of the tooth. The processor 103 may adjust the color balance and contrast settings to highlight specific features or anomalies on the tooth surface based on the mirror's orientation.

Automatically adjusting the settings based on the mirror's position reduces the need for manual adjustments by the dentist, making the examination process more efficient and user-friendly.

### Example:

In an embodiment, the processor 103 may provide real-time feedback and automatically adjust the settings as the dentist moves the mirror 105, ensuring optimal performance without interrupting the examination. The system may store and recall preferred settings for specific positions, allowing for quick and easy adjustments during subsequent examinations.

By considering the location and orientation of the mirror 105, the dental examination system 100 can provide optimal illumination, enhanced image quality, accurate diagnosis, and improved user convenience, ultimately leading to better patient outcomes.

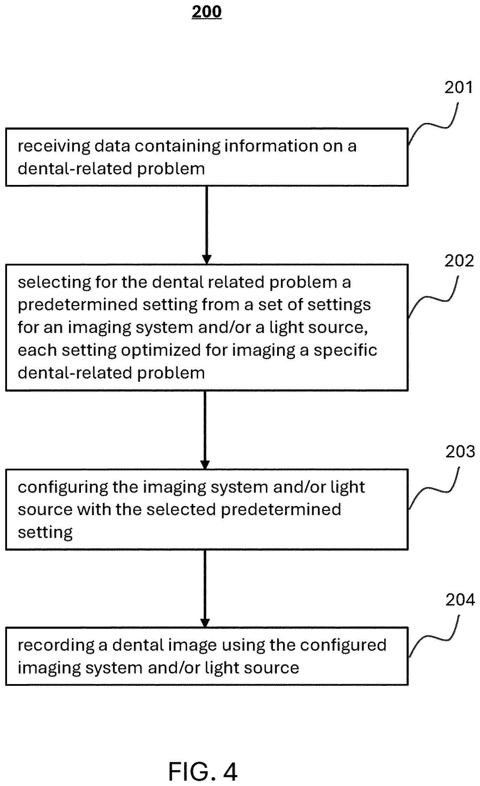

In a second aspect of the invention, and as illustrated in FIG 4, a method 200 for recording a dental image is presented, comprising: receiving 201 data containing information on a dental-related problem; selecting 202 for the dental related problem a predetermined setting from a set of settings for an imaging system 101 and/or a light source 102, each setting optimized for imaging a specific dental-related problem; configuring 203 the imaging system and/or light source with the selected predetermined setting; recording 204 a dental image using the configured imaging system 101 and/or light source 102.

Thus, the data is used to select which settings of the imaging system 101 and/or light source 102 are most optimal for imaging the dental problem. The settings are retrieved from a set of predetermined settings for the imaging system 101 and/or the light source 102. Each of these settings are predetermined for a particular dental related problem. Once the information/data on the dental related problem is received, the particular settings for the imaging system 101 and/or light source 102 are extracted from a set of predetermined settings and used to configure the imaging system 101 and/or the light source 102. The selection may be performed from a look-up table containing the predetermined settings. Once the imaging system 101 and/or light source 102 are configured, the imaging system 101 and light source 102 are used to record an image. The light source 102 is activated timely ensuring that imaging using the imaging system 101 can be done.

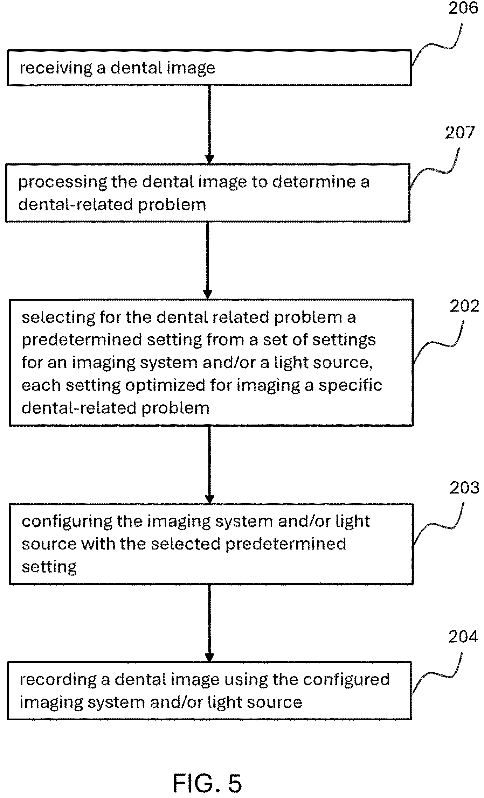

According to an embodiment and illustrated in FIG 5, receiving 201 data containing information on a dental-related problem comprises: receiving 206 a dental image; and processing 207 the dental image to determine a dental-related problem.

According to an embodiment, the method 200 may further comprise selecting the predetermined setting based on wavelength selection and/or filter characteristics of the imaging system and/or light source.

According to an embodiment, the method 200 may further comprise selecting the predetermined setting based on the location and/or orientation of a mirror relative to a tooth surface, wherein the mirror integrates the imaging system and the light source.

According to an embodiment, the method 200 may further comprise adapting one or more settings of the imaging system 101 and/or light source 102, selected from auto-zoom, contrast, frame rates, resolution, and light intensity, based on the received data. Settings of the imaging system 101 may be: auto-zoom, contrast, frame rates, resolution. Settings of the light source 102 may be: light intensity.

## Claims

1. A dental examination system 100, comprising:
an imaging system 101 for recording a dental image;
a light source 102;
a processor 103 configured to:
receive data containing information on a dental related problem;
select for the dental related problem a predetermined setting from a set of settings for the imaging system and/or the light source, each setting optimized for imaging a specific dental-related problem;
configure the imaging system 101 and/or light source 102 with the selected predetermined setting;
recording a dental image using the configured imaging system 101 and/or light source 102.

2. The system 100 according to claim 1, wherein the data is received from a source 104 external to the system 100.

3. The system 100 according to claim 1, wherein receiving data containing information on a dental related problem comprises:
receiving the dental image; and
processing the dental image to determine the dental related problem.

4. The system 100 according to any of the preceding claims, the set of settings comprises wavelength selection and/or filter characteristics of the imaging system 101 and/or light source 102.

5. The system 100 according to any of the preceding claims, further comprising an oral healthcare device configured to generate the data, preferably a toothbrush.

6. The system 100 according to any of the preceding claims, wherein the system 100 is a portable dental examination instrument further comprising a mirror 105 and wherein the processor 103 is further configured to select the predetermined setting based on location and/or orientation of the mirror 105, preferably relative to a tooth surface.

7. The system 100 of claim 6, wherein the mirror integrates the imaging system 101 and the light source 102.

8. The system 100 according to any of the preceding claims, wherein the processor 103 is configured to adapt one or more settings of the imaging system 101 and/or the light source 102 such as auto-zoom, contrast, frame rates, resolution, and light intensity, based on the data.

9. A method 200 for recording a dental image, comprising:
receiving 201 data containing information on a dental-related problem;
selecting 202 for the dental related problem a predetermined setting from a set of settings for an imaging system 101 and/or a light source 102, each setting optimized for imaging a specific dental-related problem;
configuring 203 the imaging system 101 and/or light source 102 with the selected predetermined setting;
recording 204 a dental image using the configured imaging system 101 and/or light source 102.

10. The method of claim 9, further comprising: receiving 205 the data from a source external to the smart dental mirror system.

11. The method of any of claim 9 to 10, wherein receiving 201 data containing information on a dental-related problem comprises:
receiving 206 a dental image; and
processing 207 the dental image to determine the dental-related problem.

12. The method of any of claims 9 to 11, further comprising: selecting the predetermined setting based on wavelength selection and/or filter characteristics of the imaging system 101 and/or light source 102.

13. The method of any of claims 9 to 12, further comprising: selecting the predetermined setting based on the location and/or orientation of a mirror 105 relative to a tooth surface, wherein the mirror 105 integrates the imaging system 101 and the light source 102.

14. The method of any of claims 9 to 13, further comprising: adapting one or more other settings of the imaging system 101 and/or light source 102 selected from: auto-zoom, contrast, frame rates, resolution, and light intensity, based on the received data.

15. The method of any of claims 9 to 14, wherein the method is implemented in the processor 103 of the system 100 according to any of claims 1 to 8.
